# DEMANDE DE BREVET EUROPEEN

(11) **EP 1 477 158 A1**
(43) Date de publication de la demande: **17.11.2004**
(21) Numéro de dépôt: 04291139.6
(22) Date de dépôt: 04.05.2004
(51) Int. Cl.: A61K 7/09, A61K 7/06

(54) **Dithiols et leur utilisation pour le renforcement du cheveu**

(30) Priorité: 16.05.2003 FR 0305899
(71) Demandeur: L'OREAL, 75008 Paris (FR)
(72) Inventeur: Philippe, Michel, 91320 Wissous (FR); Andrean, Hervé, 75014 Paris (FR); Barbarat, Philippe, 92270 Bois-Colombes (FR)
(74) Mandataire: Bourdeau, Françoise

(57) **Abrégé**

L'invention a pour objet l'utilisation en cosmétique de dithiols de structure particulière, pour le renforcement des matières kératiniques. Elle vise également de nouveaux dithiols ainsi que des compositions cosmétiques les contenant.

## Description

L'invention a pour objet l'utilisation en cosmétique de dithiols de structure particulière, pour le renforcement des matières kératiniques. Elle vise également de nouveaux dithiols ainsi que des compositions cosmétiques les contenant.

Le terme "matière kératinique" englobe la peau et les fibres kératiniques. On entend par *"fibres kératiniques",* les cheveux, les cils, les sourcils, les poils et notamment les cheveux. La présente invention vise en particulier les fibres kératiniques.

Les composés à fonction thiol sont connus et utilisés en cosmétique comme réducteurs dans la mise en forme durable du cheveu, spécialement en permanente. Ces composés comme l'acide thioglycolique et ceux brevetés par exemple dans les brevets US 2, 719,813, US 2, 719,814 et récemment, dans la demande W001/74318, présentent cependant des désavantages liés à une dégradation de la fibre.

Par ailleurs, il est déjà connu, en cosmétique, l'utilisation de certains dithiols. En particulier, la demande de brevet français, déposée par la Demanderesse sous le numéro 0204 352 et, non encore publiée, divulgue un procédé de gainage métallique conférant, aux fibres kératiniques, des propriétés cosmétiques rémanentes aux shampooings. Selon ce procédé, on applique sur les fibres kératiniques une composition contenant au moins un sel métallique, puis on applique une composition contenant au moins un agent réducteur. Parmi ces derniers sont cités les BMS (bismercaptoéthylsulfone), DTT (dithiothréitol), DMH (N,N'diméthyl-N,N'-bis(mercaptoacétyl)hydrazine).

Cette demande n° 0204 352 indique, en toutes généralités, que le procédé comprenant l'application d'un sel métallique et d'un réducteur peut être utilisé pour la fixation et/ou le maintien des cheveux, le soin des cheveux, les shampooings, le conditionnement des cheveux, par exemple pour leur apporter de la douceur ou encore pour le maquillage des cheveux. Par contre, elle ne divulgue pas l'utilisation spécifique des dithiols, pour l'augmentation de la rigidité des fibres kératiniques.

Au sens de la présente invention, on entend par *"renforcement des matières kératiniques",* une amélioration des propriétés mécaniques de ces dernières, pouvant se manifester:
- soit par une rigidification des matières kératiniques, qui leur procure davantage de consistance et de corps, ainsi qu'un toucher plus agréable. Il en résulte une augmentation du volume des fibres kératiniques ainsi qu'une facilité et un maintien du coiffage plus élevés.
- soit par une meilleure résistance à des forces mécaniques de traction qui leur sont appliquées, par exemple lors d'un peignage, en particulier sur des cheveux africains ou tous cheveux fragilisés ou sensibilisés. Pour imager, on parle d'effet anti-casse.

Le problème posé par la présente invention est de fournir des dithiols produisant un renforcement des matières kératiniques, cet effet étant, en outre, si possible, rémanent après cinq shampooings.

Pour résoudre ce problème, l'invention propose l'utilisation de dithiols de structure (I) pour obtenir un renforcement des matières kératiniques et notamment des fibres kératiniques: 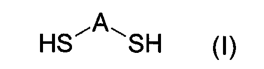
- A = chaîne hydrocarbonée en C-1 à C-30, saturée ou non, linéaire ou ramifiée pouvant être interrompue par un ou plusieurs hétéro-atomes ou fonctions tels 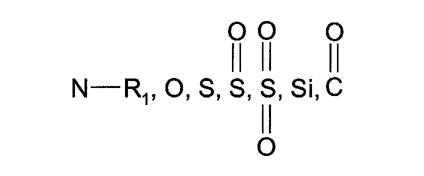
ou/et par un ou plusieurs cycles aromatiques ou non aromatiques (à 5, 6 ou 7 chaînons) et pouvant être substituée par un ou plusieurs groupements : COOH, OH, NH₂, alkyl (C-1 à C-8), alkyl (C-1 à C-8) amino, acyl (C-1 à C-8) amino, acyl (C-1 à C-8) oxy, alkyl (C-1 à C-8) oxycarbonylamino, alkyl (C-1 à C-8) amino-carbonyloxy, halogéno, alkyl (C-1 à C-8) aminocarbonyl,
- R₁ = H, alkyl (C-1 à C-8), acyl (C-1 à C-8), alkyl (C-1 à C-8) oxycarbonyl, alkyl (C-1 à C-8) amino-carbonyl, halogéno,
- A peut être également représenté par un cycle aromatique ou non aromatique à 5, 6 ou 7 chaînons, éventuellement substitué par les mêmes groupements que ceux déjà indiqués pour la chaîne aliphatique.

Selon un premier mode de réalisation de l'invention, les composés de formule (I) sont utilisés pour augmenter la rigidification des matières kératiniques, et notamment des cheveux.

Selon un deuxième mode de réalisation de l'invention, les composés de formule (I) sont utilisés pour procurer aux cheveux un effet anti-casse, et notamment sur des cheveux africains ou tous cheveux fragilisés ou sensibilisés.

Un autre objet de l'invention concerne l'utilisation des dithiols de structure (II), comme composés préférés: 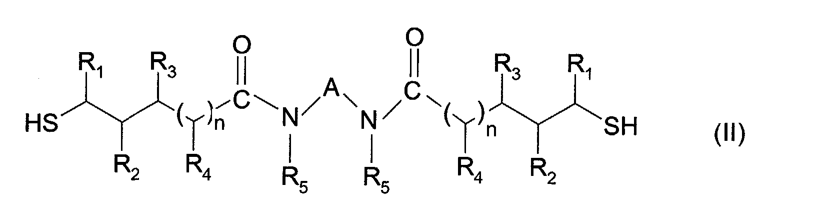 Avec • n = 0 ou 1
- A = alkylène en C-1 à C-18, saturé ou non, linéaire ou non, pouvant être interrompu par un ou plusieurs cycles aromatiques ou non aromatiques (5, 6 ou 7 chaînons) ou par un ou plusieurs hétéro-atomes tels 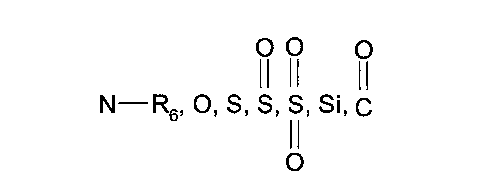 A peut également être représenté par un cycle aromatique ou non aromatique à 5, 6 ou 7 chaînons.
   A peut être substitué par un ou plusieurs groupements : COOH, OH, NH₂, alkyl (C-1 à C-8), alkyl (C-1 à C-8) amino, acyl (C-1 à C-8) amino, acyl (C-1 à C-8) oxy, alkyl (C-1 à C-8) oxycarbonylamino, alkyl (C-1 à C-8) amino-carbonyloxy, halogéno, alkyl (C-1 à C-8) aminocarbonyl.
- R₁, R₂, R₃, R₄ = H, COOH, OH, NH₂, alkyl (C-1 à C-8), alkyl (C-1 à C-8) amino, acyl (C-1 à C-8) amino, acyl (C-1 à C-8) oxy, alkyl (C-1 à C-8) oxycarbonylamino, alkyl (C-1 à C-8) amino-carbonyloxy, halogéno, alkyl (C-1 à C-8) aminocarbonyl
- R₅, R₆ = H, alkyl (C-1 à C-4), acyl (C-1 à C-4), alkyl (C-1 à C-4) oxycarbonyl, alkyl (C-1 à C-4) amino-carbonyl,.

La liste et la structure des composés préférés répondant à la formule II sont données ci-après : 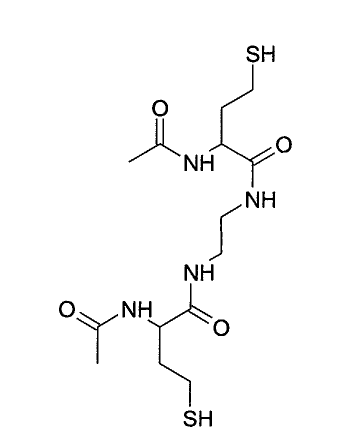
(1) 2-Acetylamino-N-[2-(2-acetylamino-4-mercapto-butyrylamino)-éthyl]-4-mercapto-butyramide 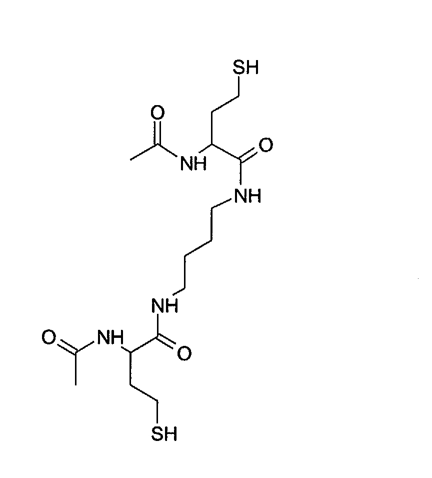
(2) 2-Acetylamino-N-[2-(2-acetylamino-4-mercapto-butyrylamino)-butyl]-4-mercapto-butyramide 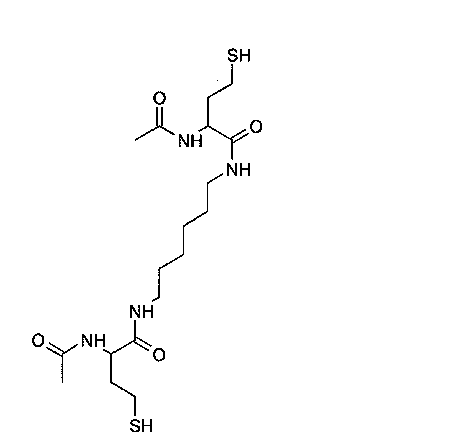
(3) 2-Acetylamino-N-[2-(2-acetylamino-4-mercapto-butyrylamino)-hexyl]-4-mercapto-butyramide 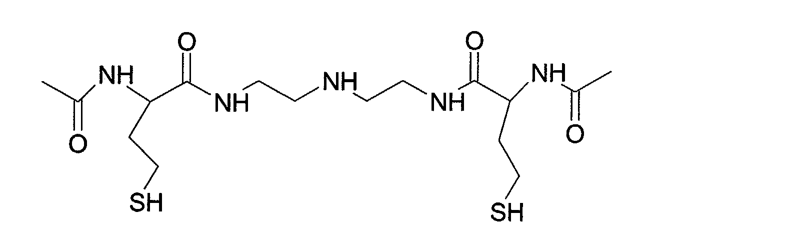
(4) 2-Acetylamino-N-{2-[2-(2-acetylamino-4-mercapto-butyrylamino)-éthylamino]-éthyl}-4-mercapto-butyramide 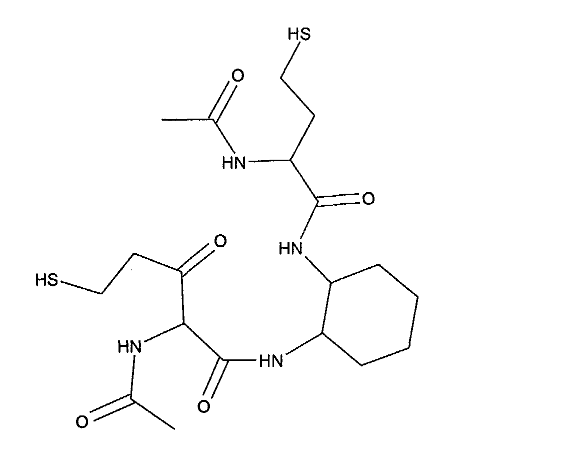
(5) 2-Acetylamino-N-[2-(2-acetylamino-4-mercapto-butyrylamino)-cyclohexyl]-4-mercapto-butyramide 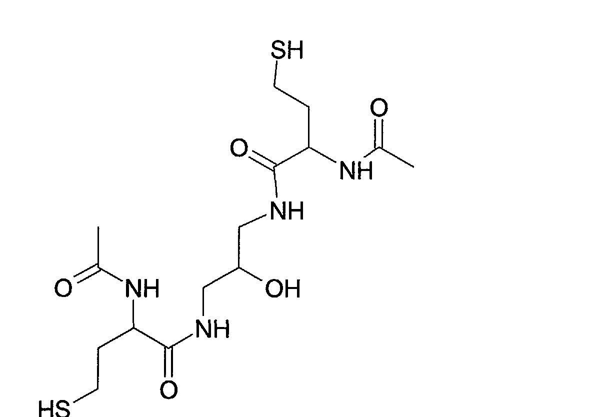
(6) 2-Acetylamino-N-[2-(2-acetylamino-4-mercapto-butyrylamino)-2-hydroxypropyl]-4-mercapto-butyramide 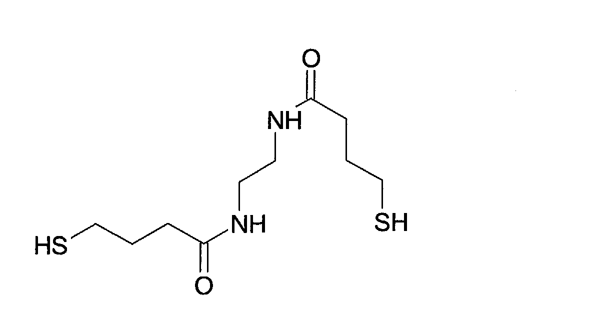
(7) 4-Mercapto-N-[2-(4-mercapto-butyrylamino)-éthyl]-butyramide

Parmi ces composés de formule II, on préfère tout particulièrement le 2-acetylamino-N-{2-[2-(2-acetylamino-4-mercapto-butyrylamino)-éthylamino]-éthyl}-4-mercapto-butyramide.

De manière générale, la synthèse des composés de formule II est réalisée comme suit : on traite sous atmosphère inerte la solution d'une thiolactone par une solution de diamine, la température de réaction est comprise entre zéro degrès Celsius et la température de reflux du solvant pour une durée de 15 minutes à 12 heures.

Un autre objet de l'invention concerne également l'utilisation des dithiols de structure (III), comme composés préférés : 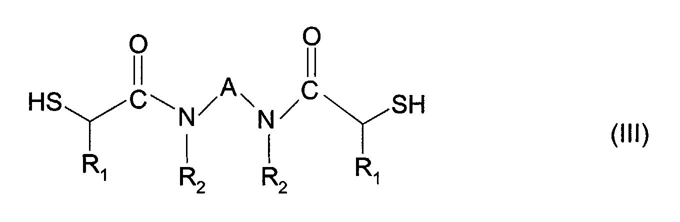
- A = alkylène en C-1 à C-18, saturé ou non, linéaire ou non, pouvant être interrompu par un ou plusieurs cycles aromatiques ou non aromatiques (5, 6 ou 7 chaînons) ou par un ou plusieurs hétéro-atomes tels 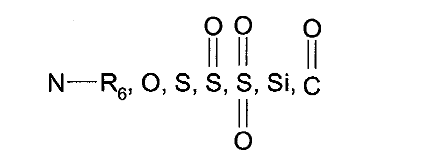 A peut également être représenté par un cycle aromatique ou non aromatique à 5, 6 ou 7 chaînons.
   A peut être substitué par un ou plusieurs groupements : COOH, OH, NH₂, alkyl (C-1 à C-8), alkyl (C-1 à C-8) amino, acyl (C-1 à C-8) amino, acyl (C-1 à C-8) oxy, alkyl (C-1 à C-8) oxycarbonylamino, alkyl (C-1 à C-8) amino-carbonyloxy, halogéno, alkyl (C-1 à C-8) aminocarbonyl.
- R₁, R₂ = H, alkyl C-1 à C-8.

Encore un autre objet de l'invention concerne une composition cosmétique comprenant, dans un milieu cosmétiquement acceptable, au moins un dithiol de formule (II).

La composition de l'invention peut être à usage cosmétique ou pharmaceutique. Préférentiellement, la composition de l'invention est à usage cosmétique. Aussi, la composition doit contenir un milieu physiologiquement acceptable non toxique et susceptible d'être appliqué sur la peau, les phanères ou les lèvres d'êtres humains. Par "cosmétique", on entend au sens de l'invention une composition d'aspect, d'odeur et de toucher agréables.

En particulier, la composition à application sur le cuir chevelu ou les cheveux peut se présenter sous forme d'une lotion de soin capillaire, par exemple d'application journalière ou bi-hebdomadaire, d'un shampooing ou d'un après-shampooing capillaire, en particulier d'application bi-hebdomadaire ou hebdomadaire, d'un savon liquide ou solide de nettoyage du cuir chevelu d'application journalière, d'un produit de mise en forme de la coiffure (laque, produit pour mise en pli, gel coiffant), d'un masque traitant, d'une crème ou d'un gel moussant de nettoyage des cheveux. Elle peut encore se présenter sous forme de teinture ou de mascara capillaire à appliquer au pinceau ou au peigne.

Avantageusement, la composition de l'invention comprend au moins un dithiol (1) à (7).

Les compositions selon l'invention peuvent également contenir, de préférence, des additifs cosmétiques et/ou des adjuvants de formulation tels que, en particulier, les agents tensioactifs anioniques, cationiques, amphotères ou non-ioniques, les agents nacrants, les agents opacifiants, les pigments et colorants, les huiles, les cires dont les céramides, les filtres UV organiques ou minéraux, les agents anti-radicalaires, les vitamines, les protéines, les agents anti-pelliculaires, les agents plastifiants, les agents d'ajustement et de fixation du pH, les agents anti-oxydants, les agents conservateurs, les précurseurs de colorants capillaires et les agents oxydants.

Ces compositions peuvent être conditionnées dans un dispositif aérosol en présence d'un ou de plusieurs agents propulseurs. Ces agents propulseurs sont de préférence choisis parmi le diméthyléther, les alcanes en C₃₋₅, le 1,1-difluoroéthane, les mélanges de diméthyléther et d'alcanes en C₃₋₅ et les mélanges de 1,1-difluoroéthane et de diméthyléther et/ou d'alcanes en C₃₋₅.

L'homme de métier veillera à choisir les éventuels additifs et leur quantité de manière à ce qu'ils ne nuisent pas aux propriétés intéressantes des compositions de la présente invention.

Les compositions peuvent se présenter sous la forme de fluides épaissis, de gels, de crèmes, de mousses, d'émulsions E/H, H/E ou d'émulsions multiples.

Les compositions de l'invention peuvent être appliquées sur les cheveux, la peau, les cils, les sourcils et les ongles.

L'invention va être plus complètement illustrée à l'aide de l'exemple non limitatif suivant.

### Exemple:

On réalise six composés répondant à la formule II. Ces composés ont été caractérisés par RMN du proton, spectrométrie de masse et dosage de la fonction thiol. Ces composés sont tous réalisés à partir d'une amine et de N-acétylthiolactone de l'homocystéine ou d'une amine et de butyrothiolactone, selon un mode opératoire similaire.

### Synthèse du 2-Acetylamino-N-[2-(2-acetylamino-4-mercapto-butyrylamino)-éthyl]-4-mercapto-butyramide (1)

On additionne sous atmosphère d'argon la solution d'éthylènediamine dans le dichlorométhane (10⁻² mole dans 15 ml) à la solution de N-acétylthiolactone de l'homocystéine dans le dichlorométhane (2 10⁻² mole dans 35 ml), sous agitation magnétique et à température ambiante en 10 minutes. Au bout de deux heures, le milieu réactionnel se prend en masse, on porte alors deux heures au reflux du solvant, on refroidit et on filtre le précipité. On lave avec du dichlorométhane, on essore le précipité le plus possible et on le sèche à l'évaporateur rotatif on obtient 3.5 grammes de poudre blanche, dépourvue d'odeur.
Masse théorique 3.79; rendement 93 %

| Dithiol de formule II cité dans la description sous le numéro | Amine | N-acétylthiolactone de l'homocystéine | Rendement R(%) |
|---|---|---|---|
| (2) | 1-4 diaminobutane 3.10⁻² mole | 6.10⁻² mole | 91,3 |
| (3) | 1-6 diaminohexane 3.10⁻² mole | 6.10⁻² mole | 92,5 |
| (4) | Diéthylènetriamine 0.18 mole | 0.36 mole | 99 |
| (5) | Trans-1,2 diaminocyclohexane 0.1 mole | 0.2 mole | 74 |
| (6) | 1-3 diaminopropanol 5.10⁻² mole | 0.1 mole | 93.5 |

| Dithiol de formule II cité dans la description sous le numéro | Amine | Butyrothiolactone | R % |
|---|---|---|---|
| (7) | Ethylènediamine 5.10^{- 2} mole | 0.1 mole | 81 |

On introduit chaque dithiol (1) à (7) dans une composition cosmétique comprenant de 1 % à 35%, et de préférence de 5% à 15% de dithiol et de 70% à 95% d'eau. On applique les compositions sur différents types de cheveux et de préférence on procède après l'application de la composition à une étape de fixation connue. Un panel d'experts a jugé le produit traité, renforcé par rapport à un produit non traité, et dont le renforcement est encore détectable après 5 shampooings.

## Revendications

1. Utilisation de dithiols de structure (I) pour obtenir un renforcement des matières kératiniques et notamment des fibres kératiniques: 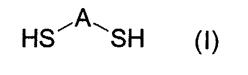
• A = chaîne hydrocarbonée en C-1 à C-30, saturée ou non, linéaire ou ramifiée pouvant être interrompue par un ou plusieurs hétéro-atomes ou fonctions tels 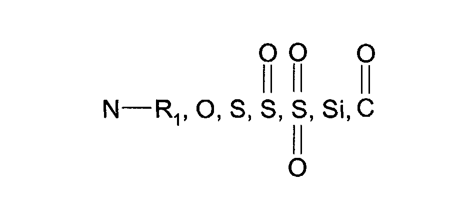
ou/et par un ou plusieurs cycles aromatiques ou non aromatiques (à 5, 6 ou 7 chaînons) et pouvant être substituée par un ou plusieurs groupements : COOH, OH, NH₂, alkyl (C-1 à C-8), alkyl (C-1 à C-8) amino, acyl (C-1 à C-8) amino, acyl (C-1 à C-8) oxy, alkyl (C-1 à C-8) oxycarbonylamino, alkyl (C-1 à C-8) amino-carbonyloxy, halogéno, alkyl (C-1 à C-8) aminocarbonyl,
• R₁ = H, alkyl (C-1 à C-8), acyl (C-1 à C-8), alkyl (C-1 à C-8) oxycarbonyl, alkyl (C-1 à C-8) amino-carbonyl, halogéno,
• A peut être également représenté par un cycle aromatique ou non aromatique à 5, 6 ou 7 chaînons, éventuellement substitué par les mêmes groupements que ceux déjà indiqués pour la chaîne aliphatique.

2. Utilisation de dithiols de structure (II) pour obtenir un renforcement des matières kératiniques et notamment des fibres kératiniques 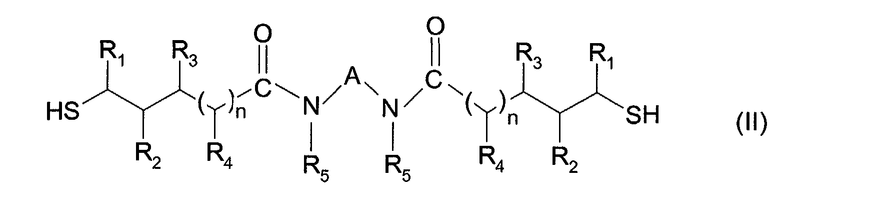 Avec
• n = 0 ou 1
• A = alkylène en C-1 à C-18, saturé ou non, linéaire ou non, pouvant être interrompu par un ou plusieurs cycles aromatiques ou non aromatiques (5, 6 ou 7 chaînons) ou par un ou plusieurs hétéro-atomes tels 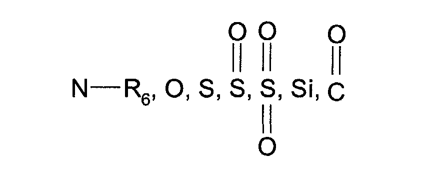 A peut également être représenté par un cycle aromatique ou non aromatique à 5, 6 ou 7 chaînons.
A peut être substitué par un ou plusieurs groupements : COOH, OH, NH₂, alkyl (C-1 à C-8), alkyl (C-1 à C-8) amino, acyl (C-1 à C-8) amino, acyl (C-1 à C-8) oxy, alkyl (C-1 à C-8) oxycarbonylamino, alkyl (C-1 à C-8) amino-carbonyloxy, halogéno, alkyl (C-1 à C-8) aminocarbonyl.
• R₁, R₂, R₃, R₄ = H, COOH, OH, NH₂, alkyl (C-1 à C-8), alkyl (C-1 à C-8) amino, acyl (C-1 à C-8) amino, acyl (C-1 à C-8) oxy, alkyl (C-1 à C-8) oxycarbonylamino, alkyl (C-1 à C-8) amino-carbonyloxy, halogéno, alkyl (C-1 à C-8) aminocarbonyl
• R₅, R₆ = H, alkyl (C-1 à C-4), acyl (C-1 à C-4), alkyl (C-1 à C-4) oxycarbonyl, alkyl (C-1 à C-4) amino-carbonyl, halogéno.

3. Utilisation pour obtenir un renforcement des matières kératiniques et notamment des fibres kératiniques de dithiols répondant à la formule II choisis parmi: 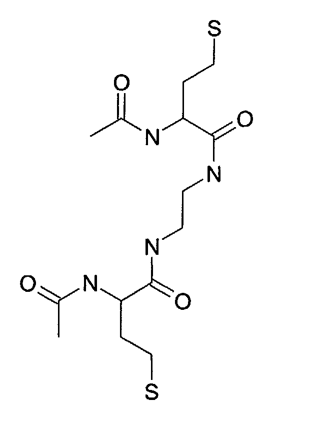
(1 ) 2-Acetylamino-N-[2-(2-acetylamino-4-mercapto-butyrylamino)-éthyl]-4-mercapto-butyramide 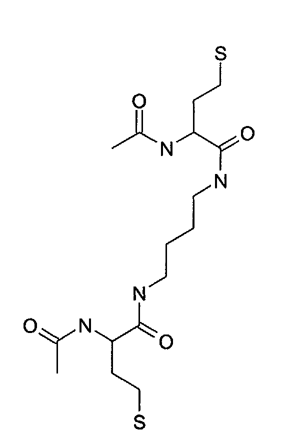
(2) 2-Acetylamino-N-[2-(2-acetylamino-4-mercapto-butyrylamino)-butyl]-4-mercapto-butyramide 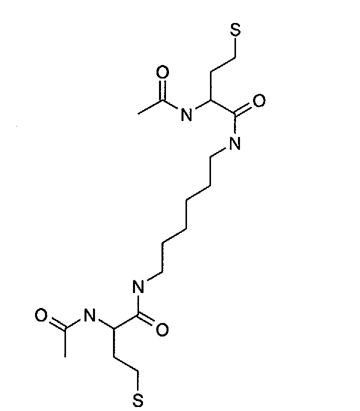
(3) 2-Acetylamino-N-[2-(2-acetylamino-4-mercapto-butyrylamino)-hexyl]-4-mercapto-butyramide 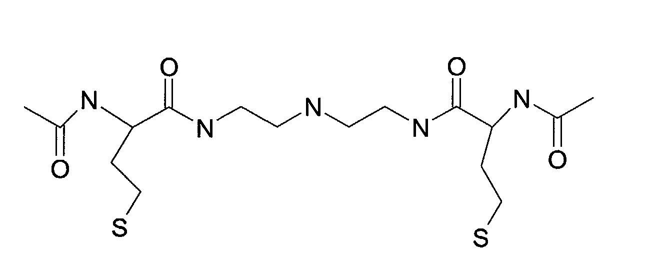
(4) 2-Acetylamino-N-{2-[2-(2-acetylamino-4-mercapto-butyrylamino)-éthylamino]-éthyl}-4-mercapto-butyramide 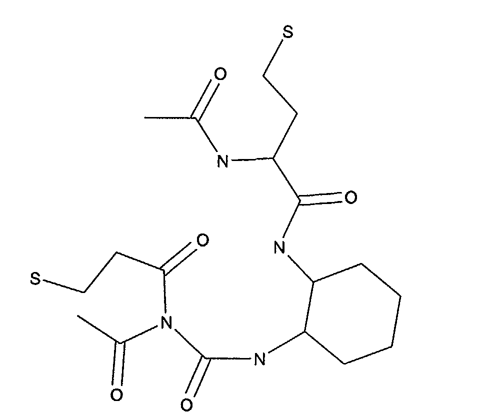
(5) 2-Acetylamino-N-[2-(2-acetylamino-4-mercapto-butyrylamino)-cyclohexyl]-4-mercapto-butyramide 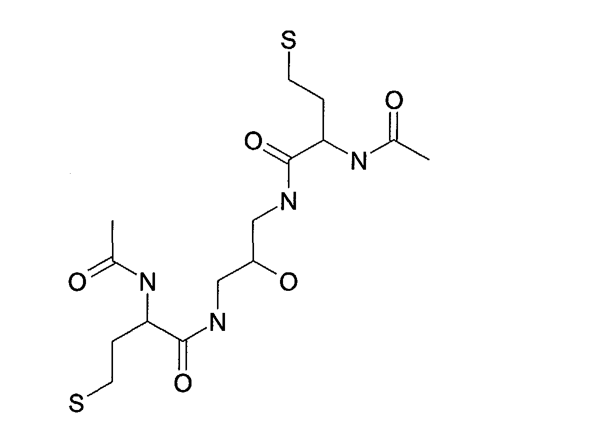
(6) 2-Acetylamino-N-[2-(2-acetylamino-4-mercapto-butyrylamino)-2-hydroxypropyl]-4-mercapto-butyramide 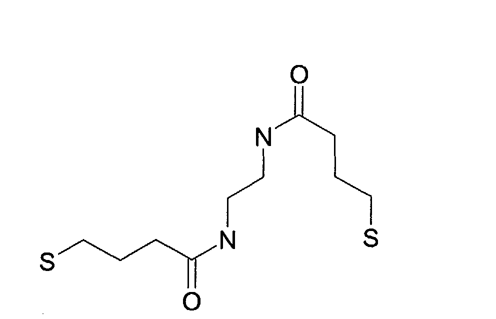
(7) 4-Mercapto-N-[2-(4-mercapto-butyrylamino)-éthyl]-butyramide

4. Utilisation de dithiols de formule II selon la revendication 2 ou 3, choisi comme étant le 2-acetylamino-N-{2-[2-(2-acetylamino-4-mercaptobutyrylamino)-éthylamino]-éthyl}-4-mercapto-butyramide.

5. Utilisation pour obtenir un renforcement des matières kératiniques et notamment des fibres kératiniques des dithiols de structure (III): 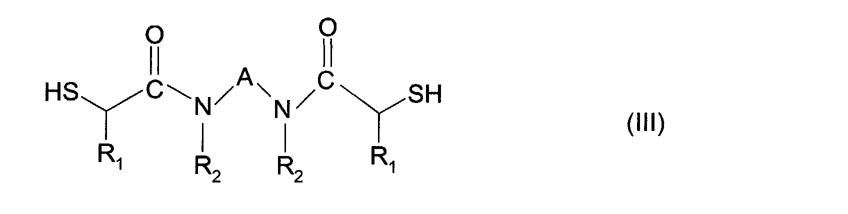
• A = alkylène en C-1 à C-18, saturé ou non, linéaire ou non, pouvant être interrompu par un ou plusieurs cycles aromatiques ou non aromatiques (5, 6 ou 7 chaînons) ou par un ou plusieurs hétéro-atomes tels 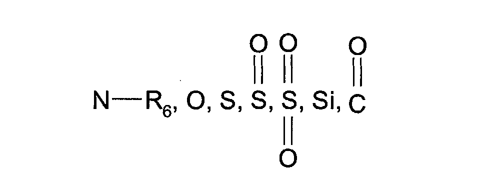 A peut également être représenté par un cycle aromatique ou non aromatique à 5, 6 ou 7 chaînons.
A peut être substitué par un ou plusieurs groupements : COOH, OH, NH₂, alkyl (C-1 à C-8), alkyl (C-1 à C-8) amino, acyl (C-1 à C-8) amino, acyl (C-1 à C-8) oxy, alkyl (C-1 à C-8) oxycarbonylamino, alkyl (C-1 à C-8) amino-carbonyloxy, halogéno, alkyl (C-1 à C-8) aminocarbonyl.
• R₁, R₂ = H, alkyl C-1 à C-8.

6. Composition cosmétique comprenant, dans un milieu cosmétiquement aceptable, au moins un dithiol de formule (II) 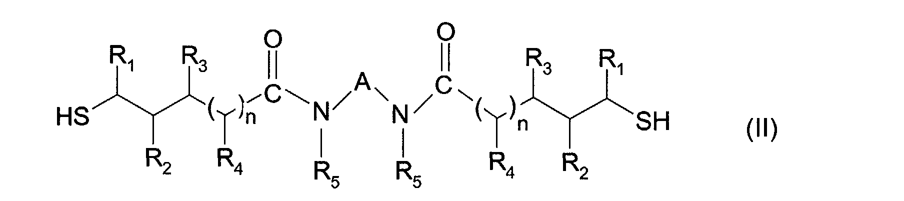 Avec
• n = 0 ou 1
• A = alkylène en C-1 à C-18, saturé ou non, linéaire ou non, pouvant être interrompu par un ou plusieurs cycles aromatiques ou non aromatiques (5, 6 ou 7 chaînons) ou par un ou plusieurs hétéro-atomes tels 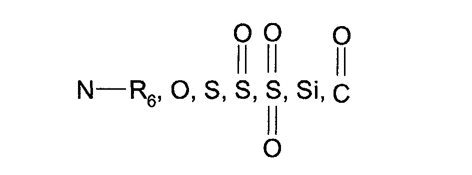 A peut également être représenté par un cycle aromatique ou non aromatique à 5, 6 ou 7 chaînons.
A peut être substitué par un ou plusieurs groupements : COOH, OH, NH₂, alkyl (C-1 à C-8), alkyl (C-1 à C-8) amino, acyl (C-1 à C-8) amino, acyl (C-1 à C-8) oxy, alkyl (C-1 à C-8) oxycarbonylamino, alkyl (C-1 à C-8) amino-carbonyloxy, halogéno, alkyl (C-1 à C-8) aminocarbonyl.
• R₁, R₂, R₃, R₄ = H, COOH, OH, NH₂, alkyl (C-1 à C-8), alkyl (C-1 à C-8) amino, acyl (C-1 à C-8) amino, acyl (C-1 à C-8) oxy, alkyl (C-1 à C-8) oxycarbonylamino, alkyl (C-1 à C-8) amino-carbonyloxy, halogéno, alkyl (C-1 à C-8) aminocarbonyl
• R₅, R₆ = H, alkyl (C-1 à C-4), acyl (C-1 à C-4), alkyl (C-1 à C-4) oxycarbonyl, alkyl (C-1 à C-4) amino-carbonyl, halogéno.

7. Composition selon la revendication 5, comprenant au moins un dithiol (1) à (7).

8. Composition selon la revendication 6 ou 7, **caractérisée en ce qu'**elle contient le dithiol (5).

9. Composition selon l'une des revendications 5 à 8, comprenant en outre des additifs cosmétiques et/ou des adjuvants de formulation tels que les agents tensioactifs anioniques, cationiques, amphotères ou non-ioniques, les agents nacrants, les agents opacifiants, les pigments et colorants, les huiles, les cires dont les céramides, les filtres UV organiques ou minéraux, les agents anti-radicalaires, les vitamines, les protéines, les agents anti-pelliculaires, les agents plastifiants, les agents d'ajustement et de fixation du pH, les agents anti-oxydants, les agents conservateurs, les précurseurs de colorants capillaires et les agents oxydants.

10. Utilisation des dithiols de structure (I) pour augmenter la rigidification des matières kératiniques, et notamment des cheveux, 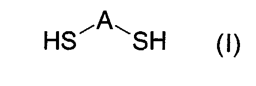
• A = chaîne hydrocarbonée en C-1 à C-30, saturée ou non, linéaire ou ramifiée pouvant être interrompue par un ou plusieurs hétéro-atomes ou fonctions tels 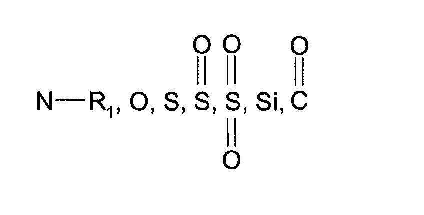 ou/et par un ou plusieurs cycles aromatiques ou non aromatiques (à 5, 6 ou 7 chaînons) et pouvant être substituée par un ou plusieurs groupements : COOH, OH, NH₂, alkyl (C-1 à C-8), alkyl (C-1 à C-8) amino, acyl (C-1 à C-8) amino, acyl (C-1 à C-8) oxy, alkyl (C-1 à C-8) oxycarbonylamino, alkyl (C-1 à C-8) amino-carbonyloxy, halogéno, alkyl (C-1 à C-8) aminocarbonyl,
• R₁ = H, alkyl (C-1 à C-8), acyl (C-1 à C-8), alkyl (C-1 à C-8) oxycarbonyl, alkyl (C-1 à C-8) amino-carbonyl, halogéno,
• A peut être également représenté par un cycle aromatique ou non aromatique à 5, 6 ou 7 chaînons, éventuellement substitué par les mêmes groupements que ceux déjà indiqués pour la chaîne aliphatique.

11. Utilisation des dithiols de formule (I) pour procurer aux cheveux un effet anti-casse, et notamment sur des cheveux africains ou tous cheveux fragilisés ou sensibilisés, 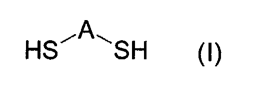
• A = chaîne hydrocarbonée en C-1 à C-30, saturée ou non, linéaire ou ramifiée pouvant être interrompue par un ou plusieurs hétéro-atomes ou fonctions tels 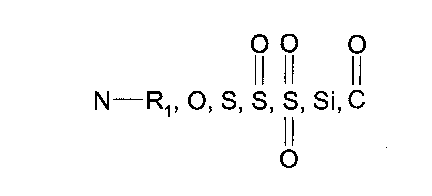 ou/et par un ou plusieurs cycles aromatiques ou non aromatiques (à 5, 6 ou 7 chaînons) et pouvant être substituée par un ou plusieurs groupements : COOH, OH, NH₂, alkyl (C-1 à C-8), alkyl (C-1 à C-8) amino, acyl (C-1 à C-8) amino, acyl (C-1 à C-8) oxy, alkyl (C-1 à C-8) oxycarbonylamino, alkyl (C-1 à C-8) amino-carbonyloxy, halogéno, alkyl (C-1 à C-8) aminocarbonyl,
• R₁ = H, alkyl (C-1 à C-8), acyl (C-1 à C-8), alkyl (C-1 à C-8) oxycarbonyl, alkyl (C-1 à C-8) amino-carbonyl, halogéno,
• A peut être également représenté par un cycle aromatique ou non aromatique à 5, 6 ou 7 chaînons, éventuellement substitué par les mêmes groupements que ceux déjà indiqués pour la chaîne aliphatique.
